(19) Europäisches Patentamt
European Patent Office
Office européen des brevets

(11) Publication number: **0 019 254**
**B1**

(12) **EUROPEAN PATENT SPECIFICATION**

(45) Date of publication of patent specification: **01.09.82**

(51) Int. Cl.³: **C 07 D 221/28,**
A 61 K 31/435

(21) Application number: **80102616.2**

(22) Date of filing: **12.05.80**

# (54) 7-Methyl and 7-methyl-8-lower alkyl B/C cis or trans morphinan-6-one compounds, process for their preparation and pharmaceutical preparations containing them.

(30) Priority: **21.05.79 US 40664**

(43) Date of publication of application:
**26.11.80 Bulletin 80/24**

(45) Publication of the grant of the patent:
**01.09.82 Bulletin 82/35**

(84) Designated Contracting States:
**AT BE CH DE FR GB IT LI NL SE**

(56) References cited:
GB - A - 2 014 142
US - A - 2 178 010
US - A - 3 654 280

TETRAHEDRON, vol. 20, 1964, pages 2247—2253, Pergamon Press IE Y. K. SAWA et al.: "Elimination of the 4-hydroxyl group of the alkaloids related to morphine-III" Page 2247

(73) Proprietor: **MILES LABORATORIES INC.**
**1127 Myrtle Street**
**Elkhart Indiana 46514 (US)**

(72) Inventor: **Kotick, Michael Paul**
**54231 Old Mill Drive**
**Elkhart Indiana 46514 (US)**
Inventor: **Polazzi, Joseph Odo**
**54226 Forest Groave**
**Elkhart Indiana 46514 (US)**
Inventor: **Leland, David Lawrence**
**523 Laurel Street**
**Elkhart Indiana 46514 (US)**
Inventor: **Schut, Robert Norman**
**67100 Ponderosa Drive**
**Edwardsburg Michigan 49112 (US)**

(74) Representative: **Dill, Erwin, Dr. et al,**
**c/o BAYER AG Zentralbereich Patente Marken und Lizenzen Bayerwerk**
**D-5090 Leverkusen 1 (DE)**

**EP 0 019 254 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

7-Methyl- and 7-methyl-8-lower alkyl B/C cis or trans morphinan-6-one compounds, process for their preparation and pharmaceutical preparations containing them

Morphine is a well known narcotic analgesic having the structural formula:

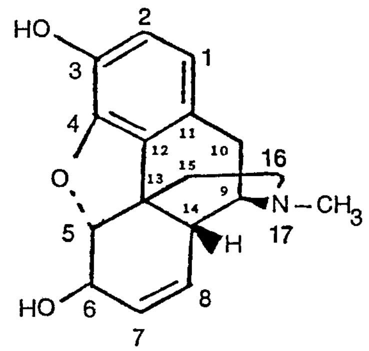

II

The compounds of this invention are structurally related to morphine and are named according to the morphinan system of nomenclature using the morphinan nucleus which is shown below:

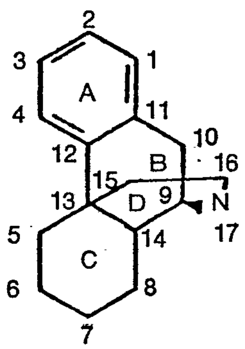

III

The numbering and stereochemical placement of atoms in the morphinan system is the same as that depicted for morphine. A dashed line is used to represent a covalent bond projecting below the plane of a reference atom while a wedged or heavily accented line signifies a covalent bond above such plane. The compounds of this invention have the same stereochemical placement of atoms as depicted for morphine unless otherwise indicated. In some structures, such as the general formula appearing on Page 1 herein, a serpentine line (~) denotes orientation of a covalent bond either above or below the plane of reference.

Another feature of the stereochemistry of the morphinan nucleus is that when the hydrogen in the 14-position is in the β-orientation the compounds have the same B/C ring junction as the naturally occurring morphine alkaloids and are referred to as the B/C *cis* isomers. Conversely, when the 14-hydrogen atom is in the α orientation the compounds are in the B/C *trans* configuration and are referred to as isomorphinans.

Morphine and its structurally related relatives are used primarily as analgesics. While extremely effective for the relief of moderate to severe pain these compounds are narcotic and most possess dependence-inducing ability and produce other side effects such as emesis, constipation, sweating, respiratory depression and myosis which make them less than ideal analgesics. It is impossible to predict, based on structure alone, whether a particular morphine-like compound will act as an analgesic (agonist), a narcotic antagonist or possess a combination of these properties since very minute structural modification in the molecule can significantly change the way it affects an individual to which it is administered. A compound with the appropriate profile of analgesic (agonist) and narcotic antagonist actions has potential for treatment of moderate to severe pain without the liability of drug dependence or drug abuse.

## PRIOR ART

The 7,8-didehydro-3-methoxy-17-methylmorphinan-6-one used as the starting material for some of the analgesic narcotic antagonists described herein is described by Sawa and Maeda (Tetrahedron *20*: 2247 [1964]), (hereinafter referred to as "SAWA").

U.S. Patent No. 3,654,280 (Y. Sawa, et al., assigned to Shionogi & Co., Ltd., published April 4, 1972; hereinafter referred to as "SHIONOGI") reports narcotic antagonist activity for certain 17-allyl, 17-dimethylallyl or 17-cyclopropylmethyl substituted 3-hydroxy-morphinan-6-one compounds related to the compound reported by SAWA.

Morphinan-6-one compounds substituted in the 8-position with an oxygen atom, as in a hydroxyl group or ether group, have been reported by Tada et al. (Tetr. Lett., 1805 [1960]). Seki (Chem. Pharm. Bull. *14*: 445 [1966]) reports a deoxytetrahydrocodeine compound substituted in the 8-position with a pyrrolidinyl group.

Small et al disclose in U.S. Patent No. 2,178,010 (issued October 31, 1939) the reaction of dihydrothebaine:

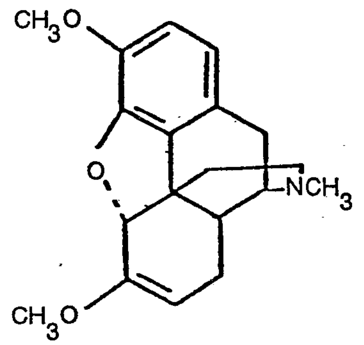

IV

with methylmagnesium iodide in refluxing ether solution for 108 hours to give, after workup which includes acid hydrolysis, a mixture from which may be isolated in 45—58% crude yield (15—17.5% recrystallized) methyldihydrothebainone:

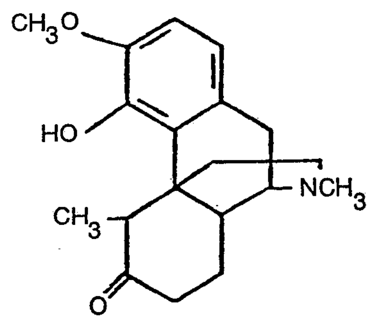

V

and a 9—11% yield (5—6% recrystallized) of isomethyldihydrothebainone:

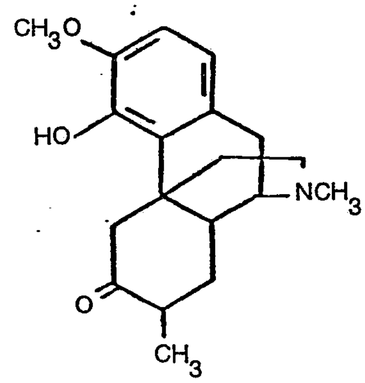

VI

Small et al. also report in J. Org. Chem., *3*, 204 (1938) the reaction of dihydrocodeinone enol acetate:

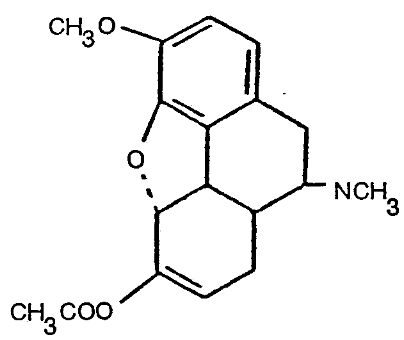

VII

with methylmagnesium iodide for 24 hours in boiling ether to give a 74% yield of V and some VI with no mention of its exact percent yield. It should be noted that the 7-methyl compound VI is the minor product of these reactions and is difficult to obtain. This is in contrast to the presently reported facile introduction of a methyl group, with concurrent 4,5-epoxy bond cleavage, into the 7-position of the morphinane nucleus.

The introduction of a 7-ketone into the morphinane nucleus with concurrent cleavage of the 4,5-epoxy bond has been reported by Rearick and Gates in Tetrahedron Letters, 507 (1970). They report that treatment of 14-bromocodeinone:

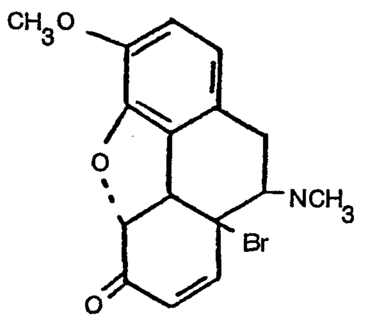

VIII

with Claisens alkali gives the 7-keto morphinane IX:

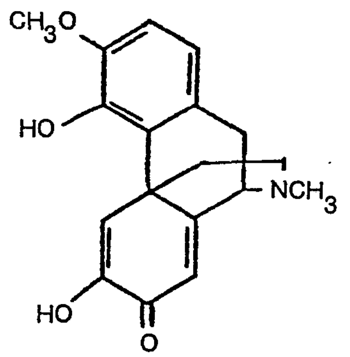

IX

Sawa et al report the preparation of desoxysinomenine characterized by the formula:

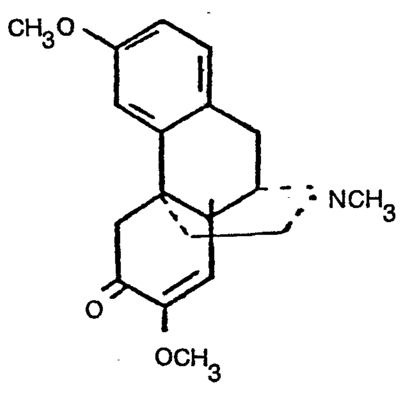

X

and desoxydihydrosinomenine characterized by the formula:

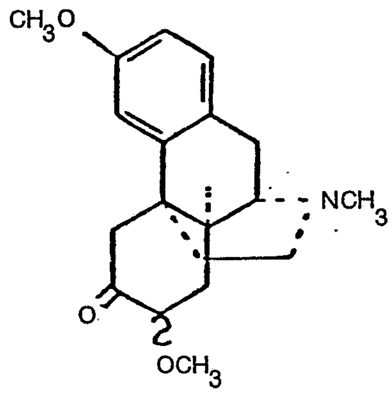

XI

in Tetrahedron, 15, 144 (1961) from the naturally occurring alkaloid, sinomenine:

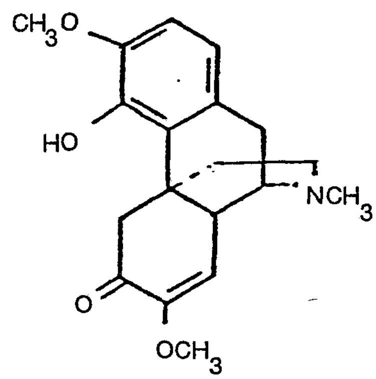

XII

Introduction of 7-substituents on the 4,5-epoxy morphinane nucleus, without cleavage of the epoxy bond has been reported by several workers. Bentley et al report in Chem. Comm., JCS C, 57 (1969) that nitrosyl chloride reacts with thebaine in methanol to give the dimethyl ketal of 7-hydroxyiminoneopinone. Reaction of thebane with iodine in the presence of $AgNO_2$ in methanol-chloroform likewise gives the dimethyl ketal of 7β-iodoneopinone.

Lester et al report in Tetrahedron, *20,* 1407 (1964) and *21,* 771 (1965) that 14-hydroxy-dihydrocodeinone may be converted to the 7-hydroxyimino derivative by reaction with amylnitrite in chloroform containing ethenolic HCl. This compound can be converted to an ethylene ketal and hydrolyzed to the 7-keto-6-ketal which upon further reaction with dimethylsulphoxonium methylide gives the oxirane.

## SUMMARY OF THE INVENTION

The present invention involves 7-methyl and 7-methyl-8-lower alkyl substituted B/C *cis* or *trans* morphinan-6-one compounds characterized by the structural formula:

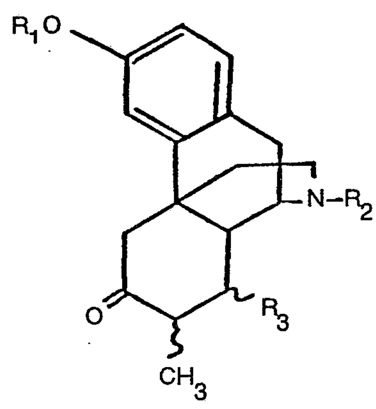

I

wherein

$R_1$ is H or methyl, $R_2$ is cyclopropylmethyl or cyclobutylmethyl and $R_3$ is H, methyl or ethyl provided that:

A. When the molecule is the B/C *cis* configuration, the 7-methyl group is in the α-configuration; and

i. when $R_2$ is cyclobutylmethyl, $R_1$ is H and $R_3$ is $\beta$-ethyl or $\beta$-methyl; and
ii. when $R_2$ is cyclopropylmethyl and $R_1$ is H, $R_3$ is H, and
iii. when $R_2$ is cyclopropylmethyl and $R_1$ is methyl, $R_3$ is $\beta$-methyl;

B. When the molecule is in the B/C *trans* configuration, the 7-methyl group is in the $\beta$-configuration, $R_1$ is H, $R_2$ is cyclopropylmethyl and $R_3$ is H or $\alpha$-methyl.

## DETAILED DESCRIPTION AND PREFERRED EMBODIMENTS

The compounds of the present invention are useful for the treatment of pain in an individual for whom such therapy is indicated. The term "individual" means a human being or an experimental animal that is a model for a human being. Due to the fact that these compounds exhibit narcotic antagonist activity in addition to their analgesic effect, they can be used for the management of pain without inducing dependence in the individual to whom they are administered. The effective dose of these compounds will vary from individual to individual but may be readily determined by one skilled in the art without undue experimentation. The compounds can be administered by any known conventional method of therapeutic administration such as intravenous, parenteral, buccal, rectal or oral. Dose forms for the administration of these compounds can be prepared by recognized methods in the pharmaceutical sciences.

The synthesis of the compounds of the present invention and their pharmacology is described in the following procedures and examples.

The preparation of those compounds corresponding to Formula I is schematically set out in Scheme I. The numbering of the compounds of Scheme I corresponds to the detailed experimental portion set out in Examples I and II. Compounds shown in Scheme I are synthesized by reaction of the first starting material thebaine *1*, a naturally occurring opium alkaloid, with lithium dimethyl cuprate. A solution of thebaine *1* in a halogenated hydrocarbon or aromatic hydrocarbon solvent is reacted with the lithium dimethyl cuprate and the reaction mixture is quenched and processed. The product, 3,6-dimethoxy-7$\beta$,17-dimethyl-4-hydroxy-5,6,8,14-tetrahydromorphinane, *2,* is isolated preferably as the crystalline chloroform solvate. This is converted to the 4-phenyl ether by reaction with bromobenzene preferably in refluxing pyridine in the presence of an acid acceptor and copper powder. The 4-phenoxy group is cleaved from *3* to give *4* by use of sodium in an appropriate solvent, preferably in a liquid ammonia-toluene mixture.

Acid hydrolysis of *4* preferably by use of 25% HCl at 100°C for one hour gives a mixture of the isomeric $\alpha,\beta$-unsaturated ketones *5* and *6* which are most conveniently separated at this stage by chromotography. Each isomer is then individually carried through the remainder of the reaction sequence.

The double bond in the 7,8-position of *5* and *6* is reduced by treatment with hydrogen over a palladium-charcoal catalyst preferably in acidified ethanol solution to give compounds *7* and *24*. In the B/C *cis* series, the hydrogen is added from the $\beta$ face of the molecule with the result that the 7-methyl group in *7* occupies the $\alpha$-position. In the B/C *trans* series we believe that the methyl group occupies the $\beta$-position.

Alternatively, *5* or *6* may be alkylated in a 1,4-manner by use of lithium dimethyl copper at about 0°C, or by use of lithium divinyl copper at about —40°, to give 7-methyl-8-alkylated-morphinan-6-ones. The 8-vinyl compound is not isolated but is reduced by catalytic hydrogenation to an ethyl group. The 17-methyl group is removed and a cycloalkylmethyl moiety introduced at this position.

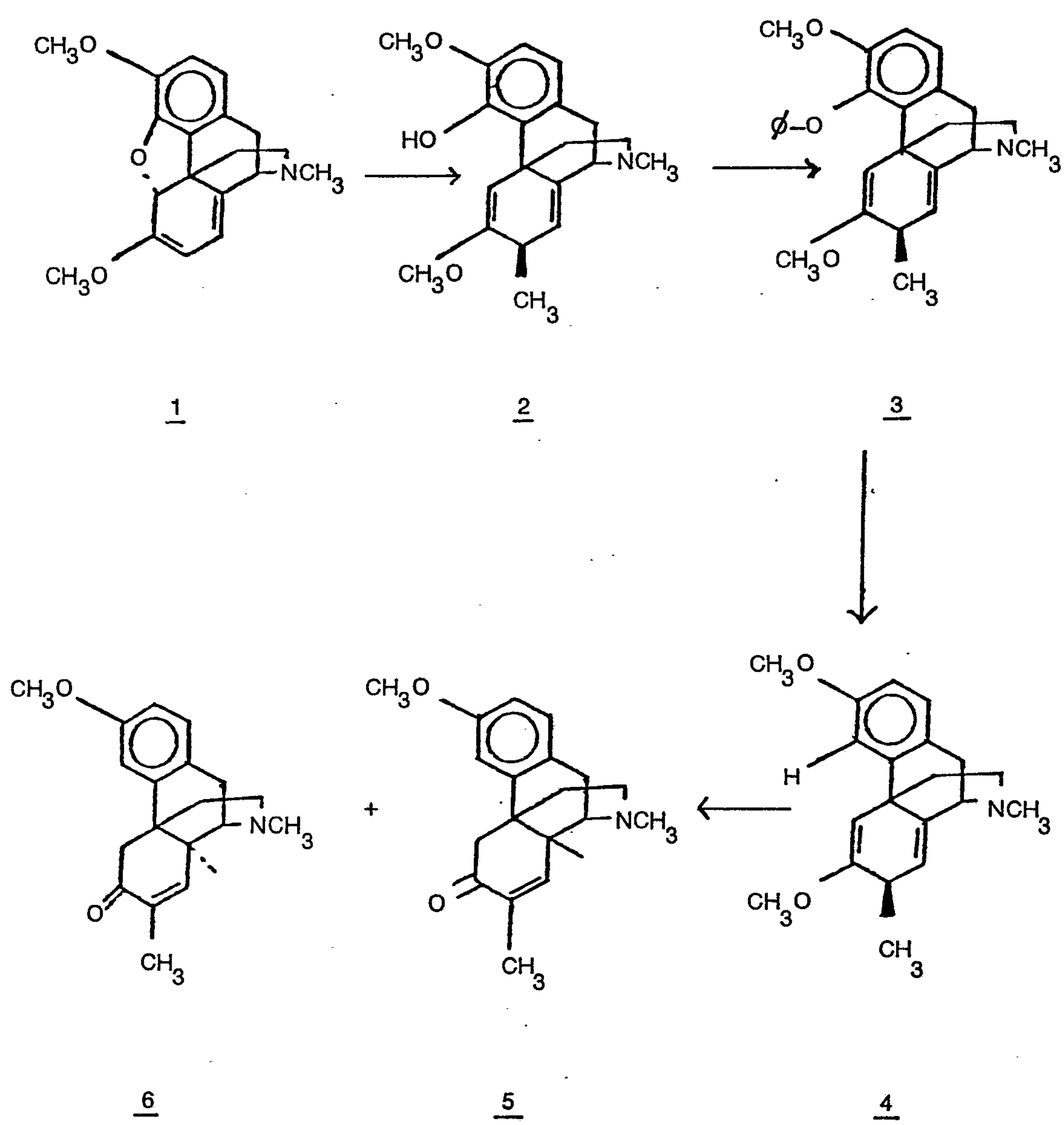

1 2 3

6 + 5 4

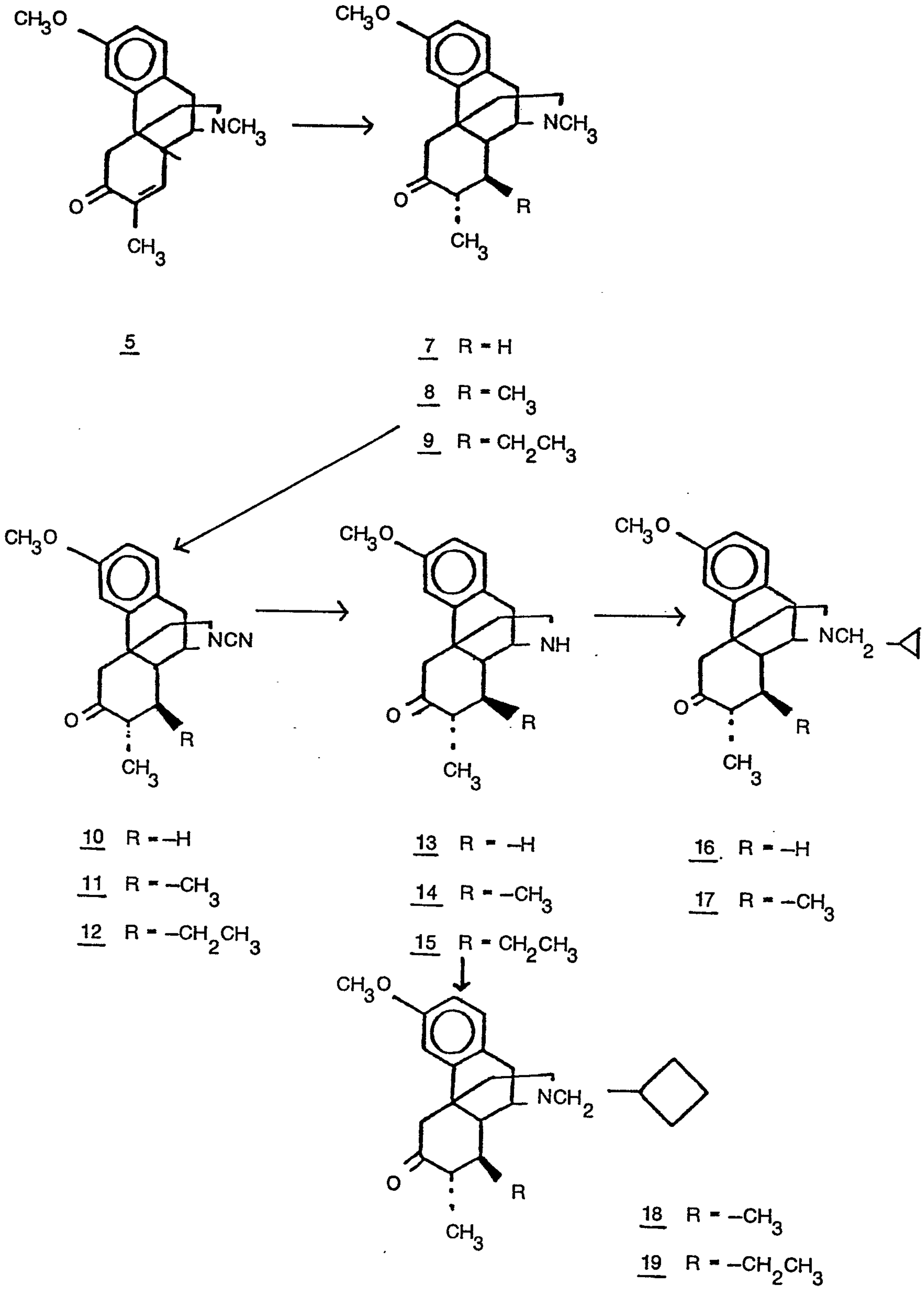

5

7 R = H

8 R = $CH_3$

9 R = $CH_2CH_3$

10 R = –H

11 R = $-CH_3$

12 R = $-CH_2CH_3$

13 R = –H

14 R = $-CH_3$

15 R = $CH_2CH_3$

16 R = –H

17 R = $-CH_3$

18 R = $-CH_3$

19 R = $-CH_2CH_3$

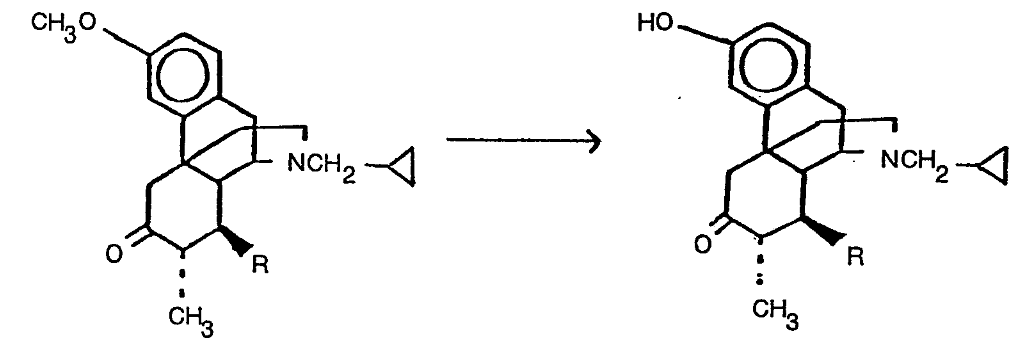

16 R = –H

17 R = $–CH_3$

20 R = –H

21 R = $–CH_3$

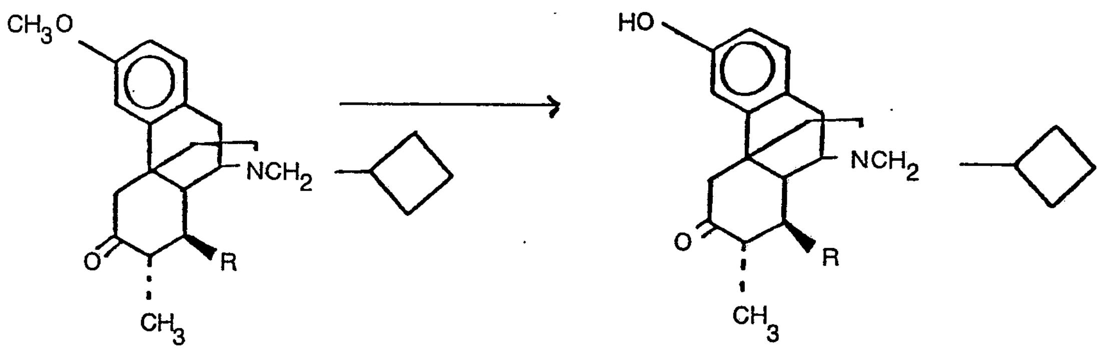

18 R = $–CH_3$

19 R = $–CH_2CH_3$

22 R = $–CH_3$

23 R = $–CH_2CH_3$

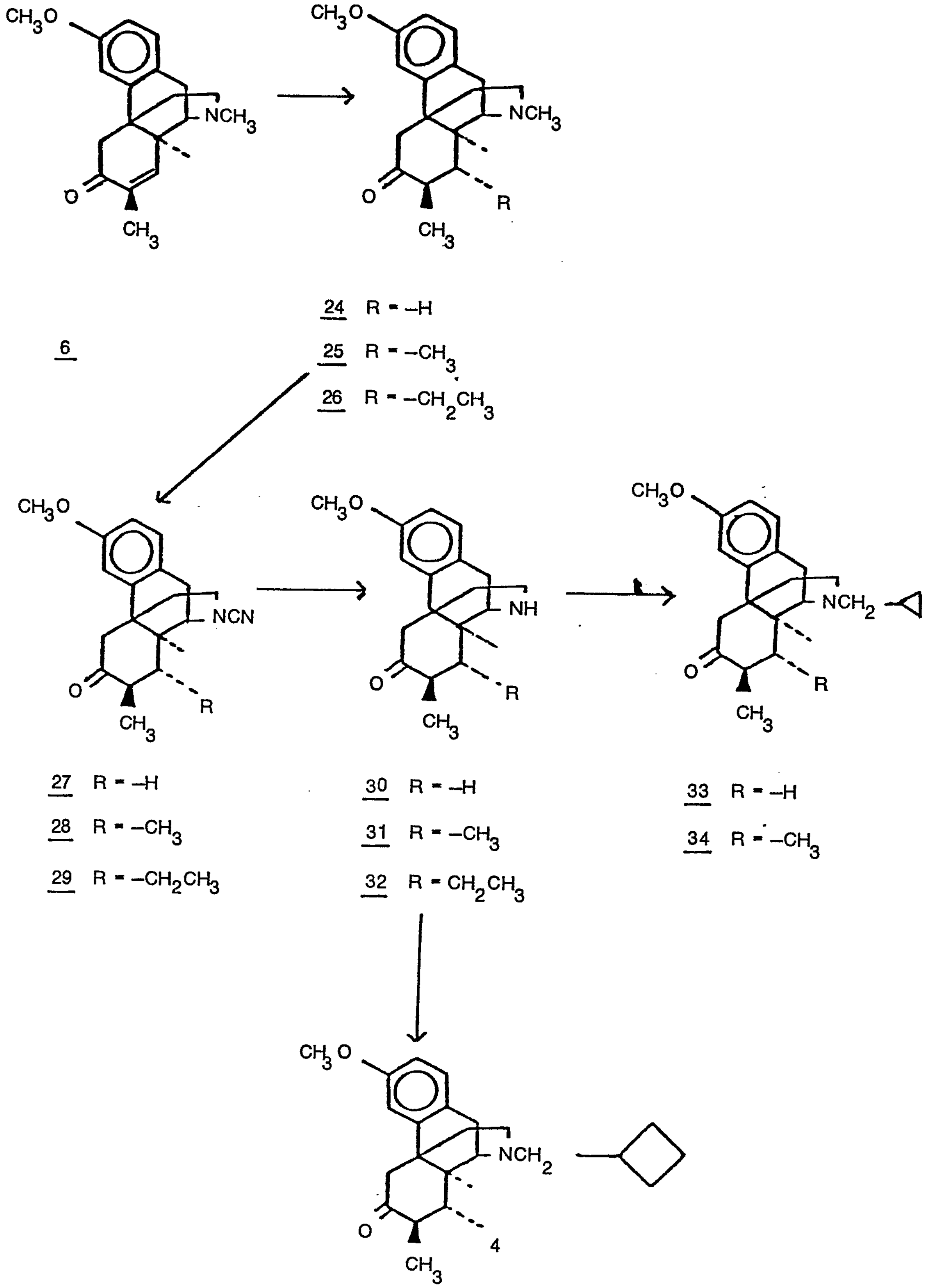

6

24 R = –H

25 R = –$CH_3$

26 R = –$CH_2CH_3$

27 R = –H

28 R = –$CH_3$

29 R = –$CH_2CH_3$

30 R = –H

31 R = –$CH_3$

32 R = $CH_2CH_3$

33 R = –H

34 R = –$CH_3$

35 R = –$CH_3$

36 R = –$CH_2CH_3$

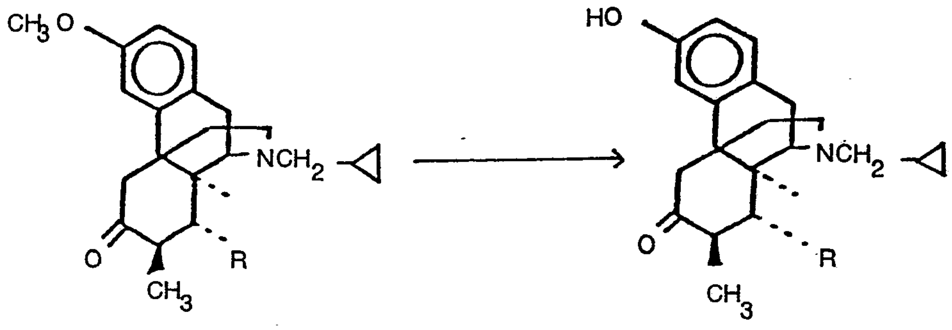

33 R = –H

34 R = $-CH_3$

37 R = –H

38 R = $-CH_3$

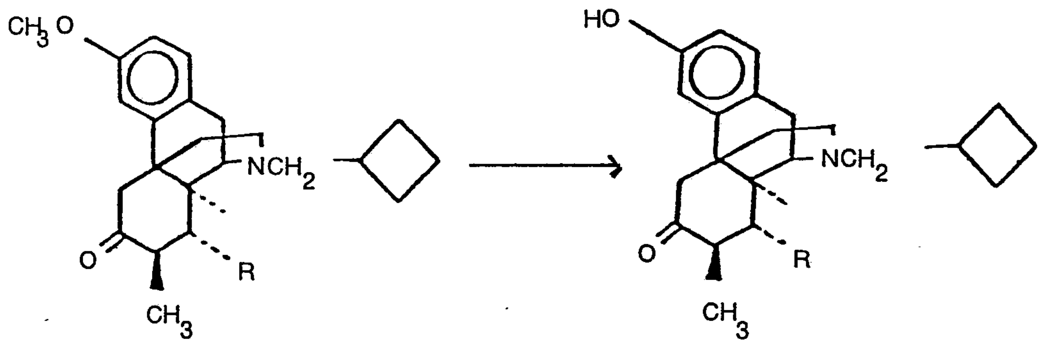

36 R = $-CH_2CH_3$

39 R = $-CH_2CH_3$

The temperatures given in the following Examples are in Centigrade.

## EXAMPLE I

### Preparation of 17-Cyclopropylmethyl and Cyclobutylmethyl-7α-Methyl-8-Unsubstituted and 8β-Alkyl-Morphinan-6-Ones

A. 3,6-Dimethyl-7β,17-dimethyl-4-hydroxy-5,6,8,14-tetradehydromorphinane (2).

To a solution of lithium dimethyl cuprate in ether (500 ml), prepared from copper iodide (23.81 g, 126 mmol) and methyl lithium (250 mmol, 136 ml of a 1.8 M solution in ether containing lithium bromide), stirred in an ice-salt bath under an argon atmosphere was added rapidly in a thin stream a solution of thebaine (*1*, 31.14 g, 100 mmol) in benzene (500 ml). The resulting suspension was stirred for 1 hr. in the cold, then poured into saturated $NH_4Cl$ solution (600 ml) and the mixture stirred for 15 min. The organic layer was separated, and the aqueous phase adjusted to pH 13—14 by use of 50% NaOH. The aqueous phase was then extracted with two portions of chloroform, the combined organic phases washed with dilute $NH_4OH$ and dried. Evaporation gave a foam which crystallized from chloroform with the addition of hexane to give 33.20 g (74%) of *2* as the mono-chloroform solvate, mp. 97—100°C. Recrystallization from the same solvent pair gave analytically pure *2*·$CHCl_3$, mp. 98—101.5°C. R [$CDCl_3$]: 3500 $cm^{-1}$ (OH). NMR ($CDCl_3$): δ 7.30, s (1), $CHCl_3$; 6.65, m(2), H1 and H2; 6.13, s(1), H5α; 5.47, d(1), H8, $J_{7,8}$=3Hz; 3.86, s(3), 3—$OCH_3$; 3.63, s(3), 6—$OCH_3$; 2.33, s(3), 17—$NCH_3$; 1.17, d(3), 7—$CH_3$, $J_{7H,7CH3}$=7Hz; exchangeable, 4—OH at ~ 6.20.

*Anal.* Calcd. for $C_{20}H_{25}NO_3 \cdot CHCl_3$ : C, 56.45; H, 5.87; N, 3.13.
Found : C, 56.27; H, 5.82; N, 3.07.

B. 3,6-Dimethoxy-7β,17-dimethyl-4-phenoxy-5,6,8,14-tetradehydromorphinane (3).

Compound *2* (18.80 g, 42 mmol) was dried by azeotropic distillation several times with pyridine and then dissolved in pyridine (100 ml). To this solution was added bromobenzene (4.90 ml, 46 mmol), powdered potassium carbonate (6.40 g, 46 mmol) and 40 μ copper powder (1.34 g, 21 mmol) and the resulting mixture refluxed under argon for 48 hours. The solution was filtered while hot, the insoluble material washed with warm pyridine and the filtrate evaporated to dryness. The residue was dissolved in benzene, treated with charcoal and evaporated to a crystalline residue. Crystallization from ethyl acetate gave 14.33 g (85%) of *3* as tan needles, mp 177—178°C. Recrystallization from ethyl acetate gave analytically pure *3*, mp 177—178°C. NMR ($CDCl_3$): δ 7.43—6.67, m(7), aromatic; 5.57, m(2), H5α and H8; 3.60, 3—$OCH_3$; 3.03, 6—$OCH_3$; 2.42, N—$CH_3$; 1.17, d(3), 7—$CH_3$, J=7Hz.

*Anal.* Calcd. for $C_{26}H_{29}NO_3$ : C, 77.39; H, 7.24; N, 3.47.
Found : C, 77.27; H, 7.16; N, 3.32.

C. 3,6-Dimethoxy-7β,17-dimethyl-5,6-8,14-tetrahydromorphinane (4).

To liquid ammonia (500 ml) at −78°C was added dropwise a soluton of *3* (17.20 g, 43 mmol) in toluene (150 ml). To the stirred biphasic system was added sodium (2.94 g, 0.128 g atoms) and the resulting blue solution stirred at −78°C for 1 hr. Excess $NH_4Cl$ was added to quench the blue color and the ammonia allowed to evaporate at room temperature. The residual suspension was diluted with 5% NaOH and extracted 3 times with ether. Evaporation of the dried organic phase gave *4* as a tan syrup. NMR ($CDCl_3$): δ 7.17—6.53, m(3), aromatic; 5.47, d(1), H8, $J_{7,8}$=4Hz; 5.10, s(1), H5α; 3.78, 3.65, 2.43, singlets; 1.23, (d(3), 7—$CH_3$, J=7Hz.

D. 7,8-Didehydro-7,17-dimethyl-3-methoxymorphinan-6-one (5) and 7,8-Didehydro-7,17-dimethyl-3-methoxy-isomorphinan-6-one (6)

A solution of *4* (2.70 g) in 25% HCl (15 ml) was heated at 90—110°C (pre-heated oil bath) for 1 hr. The cooled solution was made basic by the addition of concentrated $NH_4OH$ and extracted 3 times with chloroform. The chloroform extracts were evaporated to give a foam which was chromatographed over Silica Gel G using 10:1 chloroform-methanol. The first major fraction gave 0.89 g (35%) of crystalline material which was recrystallized from ether-ethyl acetate to give pure *5*, mp 118.5—120°C. NMR ($CDCl_3$): δ 7.27—6.67, m(4), aromatic and 118; 3.80, 2.40, singlet; 1.90, m(3), 7—$CH_3$.

*Anal.* Calcd. for $C_{19}H_{23}NO_2$ : C, 76.74; H, 7.80, N, 4.71.
Found : C, 76.76; H, 7.68; N, 4.62.

The second major fraction gave 0.93 g (36%) of *6* which was recrystallized from ethyl acetate to give an analytical sample of *6*, mp 148—150°C. NMR ($CDCl_3$): δ 7.11—6.32 (4H); 3.75, 2.45, singlets; 1.58, m(3), 7—$CH_3$.

*Anal.* Found : C, 76.78; H, 7.66; N, 4.52.

E. 7α,17-Dimethyl-3-methoxymorphinan-6-one(7).

Compound *5* (10.0 g) was hydrogenated at an initial pressure of 50 psi over 10% palladium on charcoal (1.0 g) in 95% ethanol (250 ml) containing concentrated HCl (0.5 ml) for 3 hr. After removal of the catalyst, the filtrate was evaporated to a small volume and partitioned between dilute $NH_4OH$ and chloroform. Evaporation of the chloroform gave a crystalline residue which was recrystallized from ethyl acetate-ether to give 6.90 g (69%) of pure *7*, mp 148.5—150°C. NMR ($CDCl_3$): δ 7.20—6.57, aromatic; 3.82, 2.43, singlets; 0.87, d(3), 7—$CH_3$, J=7Hz.

*Anal.* Calcd. for $C_{19}H_{25}NO_2$ : C, 76.22; H, 8.42; N, 4.68.
Found : C, 76.36; H, 8.45; N, 4.65.

F. 3-Methoxy-7α,8β,17-trimethylmorphinan-6-one (8).

To a solution of lithium dimethyl cuprate (63 mmol) prepared in ether (400 ml), under argon at ice-salt bath temperature as described in *2* above, was added *5* (15.00 g, 50 mmol) in warm benzene (350 ml) rapidly in a thin stream. The cooling bath was removed and the reaction mixture stirred at ambient temperature for 1 hr. Workup as described above gave a residue which was crystallized from ether-ethyl acetate to give 5.83 g of *8*. Chromatography of the mother liquors gave an additional 7.29 g (83% overall yield) of crystalline *8*. Recrystallization from ether gave analytically pure *8*, mp 101—102°C. NMR ($CDCl_3$): δ 7.07—6.15, aromatic; 3.77, 2.47, singlets; 1.08, unsymmetrical d(3), 8—$CH_3$; 0.88 d(3), 7—$CH_3$.

| | | | | | | | |
|---|---|---|---|---|---|---|---|
| *Anal.* Calcd. for $C_{20}H_{27}NO_2$ | : | C, | 76.64; | H, | 8.68; | N, | 4.47. |
| Found | : | C, | 76.58; | H, | 8.54; | N, | 4.34. |

G. $7\alpha$,17-Dimethyl-$8\beta$-ethylmorphinan-6-one Hydrochloride (9).

Vinyl lithium was prepared at −78°C under argon by stirring vinyl bromide (3.0 ml, 42 mmol) with t-butyllithium (63 ml of a 1.35 *M* solution in pentane) for 1 hour. The resulting suspension was transferred to a suspension of copper iodide (4.00 g, 21 mmol) in ether (100 ml) kept at −78°C. The mixture was allowed to warm to −40°C and while maintaining this temperature, a solution of *5* (5.00 g, 17 mmol) in methylene chloride (100 ml) was added rapidly dropwise. After stirring for 15 min at −40°C, the mixture was allowed to warm to 20°C and further processed as described for *2* above. Evaporation gave a residue which showed 2 major spots by thin layer chromatography for the 1,2- and 1,4-addition products. The residue was dissolved in 95% ethanol (250 ml), concentrated HCl (0.5 ml) and 10% palladium on charcoal (1.0 g) added and the mixture hydrogenated at 50 psi for 4 hr. After removal of the catalyst, the filtrate was evaporated to a small volume and partitioned between dilute $NH_4OH$ and chloroform.

The chloroform solution was evaporated to dryness and the residue chromatographed over Silica Gel G (750 g) using 15:1 chloroform-methanol containing 0.5% concentrated $NH_4OH$ as the eluant. Fractions containing the desired 1,4 product were pooled to give 2.29 g of *9* as a glass. NMR ($CDCl_3$): $\delta$ 7.07—6.53, aromatic; 3.78, 2.50, singlets, 1.43—0.70, m(6H), 7—$CH_3$ and 8—$CH_2CH_3$.

The glass was converted to *9·HCl* which was obtained as a foam for analysis.

| | | | | | | | |
|---|---|---|---|---|---|---|---|
| *Anal.* Calcd. for $C_{21}H_{29}NO{\cdot}HCl$ | : | C, | 69.31; | H, | 8.31; | N, | 3.85. |
| Found | : | C, | 69.15; | H, | 8.11; | N, | 3.59. |

H. 17-Cyano-3-methoxy-$7\alpha$-methylmorphinan-6-one (10).

To a mixture of *7* (9.90 g, 33 mmol) and potassium carbonate (6.86 g, 50 mmol) in chloroform (75 ml) was added dropwise a solution of cyanogen bromide (4.30 g, 40 mmol) in chloroform (50 ml). Upon completion of the addition, the mixture was refluxed for 90 minutes. The cooled suspension was filtered and the filtrate evaporated to dryness and azeotroped with ethanol to give 7.24 g (71%) of crystalline *10,* mp 188—192°C.

I. 17-Cyano-$7\alpha,8\beta$-dimethyl-3-methoxymorphinan-6-one (11).

To a solution of *8* (13.30 g, 42 mmol) in chloroform (125 ml) was added potassium carbonate (8.80 g, 67 mmol) followed by the dropwise addition of a solution of cyanogen bromide (5.40 g, 51 mmol) in chloroform (75 ml). The mixture was refluxed for 90 minutes, cooled and filtered from insoluble material. The filtrate was evaporated to dryness and the residue azeotroped several times with ethanol to give crystals. The crystals were boiled with ethanol and collected after cooling to give 10.40 g (75%) of *11* as white needles, mp 159—161.5°C.

J. 17-Cyano-$8\beta$-ethyl-3-methoxy-$7\alpha$-methylmorphinan-6-one (12).

To a mixture of *9* (11.30 g, 34.5 mmol) and potassium carbonate (7.15 g, 51.8 mmol) in chloroform (75 ml) was added dropwise cyanogen bromide (4.45 g, 42.0 mmol) in chloroform. The reaction mixture was refluxed for 2 hours and processed as above to give 10.6 g (86%) of *12* as a foam after azeotroping with EtOH. The foam was used without further characterization in the hydrolysis reaction.

K. 3-Methoxy-$7\alpha$-methylmorphinan-6-one Hydrochloride (13).

A suspension of *10* (7.2 g, 23 mmol) in 2 *N* HCl was refluxed for 5 hours. The clear solution was evaporated to give 7.4 g of *13* as a foam, homogeneous by thin layer chromatography, which was used without further purification in the alkylation reactions as described below.

L. $7\alpha,8\beta$-Dimethyl-3-methoxymorphinan-6-one Hydrochloride (14)

Compound *11* (11.2 g, 34.5 mmol) in 2 *N* HCl (200 ml) was refluxed for 6 hours. The solution was evaporated to a crystalline residue which was triturated with ethanol and collected to give 6.8 g (61% of *14,* mp 235—240°C.

M. $8\beta$-Ethyl-3-methoxy-$7\alpha$-methylmorphinan-6-one Hydrochloride (15).

Compound *12* (10.6 g, 32.4 mmol) and 2 *N* HCl (200 ml) was refluxed for 5 hours and the resulting solution evaporated to dryness. Upon coevaporation with ethanol, there was obtained 6.9 g (63%) of crystalline *15,* mp > 265°C.

N. 17-Cyclopropylmethyl-3-methoxy-$7\alpha$-methylmorphinan-6-one Hydrochloride (16).

A mixture of *13* (4.80 g, 15 mmol), sodium bicarbonate (2.76 g, 32.8 mmol) and cyclopropylmethyl bromide (3.02 g, 22.5 mmol) in DMF (50 ml) was heated at 100°C under an argon atmosphere for 16 hours. The suspension was filtered, the filtrate evaporated and the residue par-

titioned between dilute $NH_4OH$ and benzene. The aqueous phase was extracted twice more with benzene and the combined organic phases dried, filtered and evaporated to a glassy residue. This residue was purified by chromatography to give 3.35 g (66%) of the pure free base of *16* as a glass. NMR ($CDCl_3$): $\delta$ 7.10—6.53, m(3), H1, H2, H4,; 3.80, s(3)—$OCH_3$; 0.85, d(3), 7—$CH_3$, J=7Hz.

The HCl salt was prepared by dissolving the free base in ethanol and adding excess concentrated HCl. Evaporation followed by azeotroping with benzene gave a foam which was crystallized and recrystallized from methanol-ethyl acetate to give white, crystalline *16*, mp >265°C.

| | | | | | | | |
|---|---|---|---|---|---|---|---|
| *Anal.* Calcd for $C_{22}H_{29}NO_2{\cdot}HCl$ | : | C, | 70.29; | H, | 8.04: | N, | 3.73. |
| Found | : | C, | 70.72; | H, | 8.19; | N, | 3.73. |

O. 17 - Cyclopropylmethyl - $7\alpha,8\beta$ - dimethyl - 3 - methoxymorphinan - 6 - one Hydrochloride (17) (TR—5188).

A mixture of *14* (5.0 g, 14.9 mmol), sodium bicarbonate (2.76 g, 32.8 mmol), cyclopropylmethyl bromide (3.02 g, 22.5 mmol) in DMF (50 ml) was heated at 100° under argon for 24 hours. Processing as above for *16* gave a syrup which was purified by column chromatography to give 1.71 g (32%) of the free base of *17*. The free base was converted to the HCl salt which was isolated as a foam for analysis.

| | | | | | | | |
|---|---|---|---|---|---|---|---|
| *Anal.* Calcd for $C_{23}H_{31}NO{\cdot}HCl$ | : | C, | 70.84; | H, | 8.27; | N, | 3.59. |
| Found | : | C, | 71.08; | H, | 8.24; | N, | 3.47. |

P. 17-Cyclobutylmethyl-$7\alpha,8\beta$-dimethyl-3-methoxymorphinan-6-one Hydrochloride (18).

Compound *14* (5.0 g), sodium bicarbonate (2.76 g), cyclobutylmethyl bromide (3.33 g) in DMF was reacted as above and processed to give a syrup which was chromatographed to give 4.24 g (78%) of the free base of *18* as a glass. The HCl salt was prepared and crystallized from ethyl acetate to give pure *18*, mp 214—218°.

| | | | | | | | |
|---|---|---|---|---|---|---|---|
| *Anal.* Calcd. for $C_{24}H_{33}NO_2{\cdot}HCl$ | : | C, | 71.35, | H, | 8.48; | N, | 3.47. |
| Found | : | C, | 71.54; | H, | 8.30; | N, | 3.46. |

Q 17-Cyclobutylmethyl-$8\beta$-ethyl-3-methoxy-$7\alpha$-methylmorphinan-6-one Hydrochloride (19).

A mixture of *15* (3.50 g), sodium bicarbonate (1.26 g), cyclobutylmethyl bromide (1.79 g) in DMF (50 ml) was heated at 100° for 48 hours. Processing as above followed by chromatography gave 2.22 g (59%) of the free base of *19* as a glass. This was converted to *19* which was obtained as a foam.

| | | | | | | | |
|---|---|---|---|---|---|---|---|
| *Anal.* Calcd for $C_{25}H_{35}NO_2{\cdot}HCl$ | : | C, | 71.83; | H, | 8.65; | N, | 3.35. |
| Found | : | C, | 71.62; | H, | 8.62; | N, | 3.52. |

R. 17-Cyclopropylmethyl-3-hydroxy-$7\alpha$-methylmorphinan-6-one Hydrochloride (20) (TR—5177).

A mixture of *16* (1.16 g, 3.1 mmol) and 48% HBr (10 ml) was refluxed for 10 minutes in a preheated oil bath (140°). The solution was cooled, diluted with water, made basic with concentrated $NH_4OH$ and extracted three times with chloroform. The combined chloroform extracts were dried, filtered and evaporated to a residue which was chromatographed to yield 0.43 g (43%) of the free base of *20* as a glass. The glass was converted to the HCl salt and crystallized from MeOH to give fine needles of *20*, mp 217—220° dec.

| | | | | | | | |
|---|---|---|---|---|---|---|---|
| *Anal.* Calcd for $C_{21}H_{27}NO_2{\cdot}HCl$ | : | C, | 69.69; | H, | 7.80; | N, | 3.87. |
| Found | : | C, | 69.64; | H, | 7.80; | N, | 3.88. |

S. 17-Cyclopropylmethyl-$7\alpha,8\beta$-dimethyl-3-hydroxymorphinan-6-one (21) (TR—5196).

Compound *17* (1.0 g, 2.6 mmol) was refluxed with 48% HBr (10 ml) for 10 minutes. After cooling the mixture was diluted with water and adjusted to pH 12 with concentrated $NH_4OH$, extracted twice with chloroform and then twice with ethyl acetate. The combined organic extracts were dried, filtered and evaporated and the residue partially purified by column chromatography. The resulting foam, which showed a faster migrating minor component together with the desired product, was further purified by preparative layer chromatography on 2 mm layers of Silica Gel G using 15:1 chloroform-methanol containing 0.75% concentrated $NH_4OH$ as the eluant. Development was repeated 5 times. The major spot was extracted from the gel by use of boiling methanol. The residue obtained on evaporation of the methanol was again column chromatographed to yield 0.26 g of *21* as a white foam.

| | | | | | | | |
|---|---|---|---|---|---|---|---|
| *Anal.* Calcd. for $C_{22}H_{29}NO_2$ | : | C, | 77.84; | H, | 8.61; | N, | 4.13. |
| Found | : | C, | 76.82; | H, | 8.61; | N, | 4.33. |

T. 17-Cyclobutylmethyl-7α,8β-dimethyl-3-hydroxymorphinan-6-one (22) (TR—5195)

Compound *16* (2.04 g, 5.6 mmol) and 48% HBr (20 ml) was refluxed for 20 minutes. Workup as above gave a foam which was chromatographed to give 1.50 g (76%) of pure *22* as a foam.

| | | | | | | | |
|---|---|---|---|---|---|---|---|
| *Anal.* Calcd. for $C_{23}H_{31}NO_2$ | : | C, | 78.15; | H, | 8.84; | N, | 3.96. |
| Found | : | C, | 78.11; | H, | 9.09; | N, | 3.87. |

U. 17-Cyclobutylmethyl-8β-ethyl-3-hydroxy-7α-methylmorphinan-6-one (23) (TR—5333)

Compound *17* (1.80 g, 4.3 mmol) and 48% HBr (20 ml) was refluxed for 15 minutes and processed in the usual fashion to give a foam. The foam was crystallized from ethanol to give 1.37 g (79%) of crystalline *23*, mp 120—122°, as the ethanol solvate. Drying gave *23*·0.75 EtOH (as indicated by nmr) which was submitted for analysis.

| | | | | | | | |
|---|---|---|---|---|---|---|---|
| *Anal.* Calcd. for $C_{24}H_{33}NO_2$·0.75 EtOH | : | C, | 76.17; | H, | 9.40; | N, | 3.48. |
| Found | : | C, | 76.18; | H, | 9.45; | N, | 3.22. |

EXAMPLE II

Preparation of 17-Cyclopropylmethyl and Cyclobutylmethyl 7β-Methyl and 7β-Methyl-8α-Lower Alkyl Isomorphinane-6-One-Compounds

A. 7β,17-Dimethyl-3-methoxy-isomorphinan-6-one (24).

A solution of *6* (10.8 g, 36.3 mmol) in 95% ethanol (250 ml) containing concentrated HCl (0.5 ml) was hydrogenated over 10% palladium on charcoal (1.0 g) at 50 psi. Removal of the catalyst followed by evaporation of the filtrate gave a foam which was partitioned between dilute $NH_4OH$ and chloroform. Several further extractions with chloroform followed by evaporation of the organic phases gave a crystalline residue which was recrystallized from ethyl acetate to give 8.7 g (81%) of *24* as white needles, mp 175—176°C. NMR ($CDCl_3$); δ 7.23—6.63, m(3), H1, H2, H4; 3.82, s(3), $—OCH_3$; 2.37, s(3), $—NCH_3$; 1.08, d(3), 7—$CH_3$, J=6Hz.

| | | | | | | | |
|---|---|---|---|---|---|---|---|
| *Anal.* Calcd. for $C_{19}H_{25}NO_2$ | : | C, | 76.22; | H, | 8.42; | N, | 4.68. |
| Found | : | C, | 76.33; | H, | 8.41; | N, | 4.66. |

B. 3-Methoxy-7β,8α,17-trimethyl-isomorphinan-6-one Hydrochloride (25).

To a solution of lithium dimethyl cuprate, prepared from copper iodide (1.20 g, 6.3 mmol) and methyl lithium (12.6 mmol), in ether (75 ml) was added compound *6* (1.50 g, 5 mmol) in benzene (75 ml). After 1 hour at 0°, workup in the usual manner followed by column chromatography gave 1.35 g (86%) of *25* free base as a foam. This was converted to the HCl salt and crystallized from ethanol-ethyl acetate to give pure *25*, mp >265°. NMR ($CDCl_3$): δ 7.20—6.57, m(3), H1, H2, H4; 3.80, s(3), $—OCH_3$; 2.70, s(3), $—NCH_3$; 1.13, d(6), 7 and 8—$CH_3$, J=6Hz.

| | | | | | | | |
|---|---|---|---|---|---|---|---|
| *Anal.* Calcd. for $C_{20}H_{27}NO_2$·HCl | : | C, | 68.65; | H, | 8.06; | N, | 4.00. |
| Found | : | C, | 68.35; | H, | 7.93; | N, | 3.83. |

C. 7β,17-Dimethyl-8α-ethyl-3-methoxy-isomorphinan-6-one Hydrochloride (26)

Vinyl lithium was prepared from vinyl bromide (3.0 ml, 42 mmol) and *t*-butyllithium (85 mmol) in ether (60 ml) by stirring for 1 hour at —78° under argon. This was transferred to a —78° suspension of copper iodide (4.00 g, 21 mmol) in ether (100 ml). The mixture was allowed to warm to —40° and while maintaining this temperature, a solution of *6* (5.0 g, 16.8 mmol) in methylene chloride (100 ml) was added dropwise. The reaction mixture was left at —40° for 15 minutes and then allowed to warm to room temperature. Workup in the usual fashion gave a foam which consisted of 2 major spots as shown by thin layer chromatography for the 1,2 and 1,4 addition products. The foam was dissolved in 95% ethanol (250 ml), concentrated HCl (0.5 ml) and 10% palladium on charcoal added and the mixture hydrogenated at 50 psi for 8 hr. Processing in the usual manner followed by chromatography gave 1.57 g (28%) of the free base of *26* as a foam. The foam was converted to the HCl salt which crystallized from methanol -ethyl acetate to give pure *26*, mp 249—250° dec. NMR ($CDCl_3$): δ 7.23—6.53, m(3), H1, H2, H4; 3.80, s(3), $—OCH_3$; 2.37, s(3), $—NCH_3$; 1.40—0.67, m(8), 7—$CH_3$, 8—$CH_2CH_3$.

| | | | | | | | |
|---|---|---|---|---|---|---|---|
| *Anal.* Calcd. for $C_{21}H_{29}NO_2$·HCl | : | C, | 69.31; | H, | 8.31; | N, | 3.85. |
| Found | : | C, | 69.02; | H, | 8.45; | N, | 3.93. |

D. 17-Cyano-7β-methyl-3-methoxy-isomorphinan-6-one (27).

To a solution of *24* (8.7 g, 29 mmol) in chloroform (100 ml) containing potassium carbonate (6.1 g, 44 mmol) was added dropwise a solution of cyanogen bromide (3.1 g, 37 mmol) in chloroform (50 ml). After completion of the addition, the mixture was refluxed for 3 hours, cooled and filtered from the

insoluble material. The filtrate was evaporated to dryness and repeatedly azeotroped with ethanol until crystals formed. The solution was cooled and 5.9 g (66%) of white crystalline *27* collected. This material was used as is in the hydrolysis step to prepare *30*.

E. 17-Cyano-7β,8α-dimethyl-3-methoxy-isomorphinan-6-one (28).

A solution of *25* (free base, 10.4 g, 33.2 mmol) in chloroform (75 ml) containing potassium carbonate (6.9 g, 50 mmol) was treated dropwise with cyanogen bromide (4.3 g, 40 mmol) in chloroform (50 ml). The mixture was refluxed for 4 hr, cooled and filtered from the insoluble material. The filtrate was evaporated to dryness and the residue chromatographed to give 4.83 g (45%) of *28* as a foam.

F. 17-Cyano-8α-ethyl-3-methoxy-7β-methyl-isomorphinan-6-one (29).

To a solution of *26* (free base, 19.7 g, 60.2 mmol) in chloroform (250 ml) containing potassium carbonate (12.5 g, 90.2 mmol) was added dropwise a solution of cyanogen bromide (7.7 g, 72.2 mmol) in chloroform (100 ml). The mixture was then refluxed for 3 hours, cooled and the insoluble material removed by filtration. The filtrate was evaporated to dryness and the residue purified by column chromatography to give 10.8 g (52%) of *29* as a foam.

G. 3-Methoxy-7β-methyl-isomorphinan-6-one (30).

A mixture of *27* (5.9 g) and 2 *N* HCl (200 ml) was refluxed overnight. The mixture was concentrated to a small volume and the residue partitioned between dilute $NH_4OH$ and chloroform. The chloroform extracts were evaporated to give 5.5 g of *30* as a glass, homogeneous by thin layer chromatography. This material was used as is in the preparation of *33*.

H. 7β,8α-Dimethyl-3-methoxy-isomorphinan-6-one (31).

A mixture of *28* (4.7 g) and 2 *N* HCl (200 ml) was refluxed overnight and processed as above. The resulting foam was chromatographed to give 2.83 g of *31* which crystallized on standing. This material was used in further reactions without additional characterization.

I. 8α-Ethyl-3-methoxy-7β-methyl-isomorphinan-6-one (32).

The N-cyano compound *29* (10.7 g) and 2 *N* HCl (400 ml) was refluxed for 24 hr. The clear solution was cooled, made basic with concentrated $NH_4OH$ and extracted with chloroform. The chloroform extracts were evaporated to give 10.0 g of *35* as a foam, homogeneous by thin layer chromatography which was used without further purification for the preparation of *36*.

J. 17-Cyclopropylmethyl-3-methoxy-7β-methyl-isomorphinan-6-one Hydrochloride (33) (TR—5205).

To a solution of *30* (2.70 g, 9.5 mmol) in DMF (50 ml) was added sodium bicarbonate (0.80 g, 9.5 mmol) and cyclopropylmethyl bromide (1.55 g, 11.4 mmol) in DMF (5 ml). The mixture was heated at 100° overnight in an argon atmosphere. The cooled solution was filtered from insoluble material and the bulk of the DMF removed under high vacuum. The residue was dissolved in dilute $NH_4OH$ and extracted with three portions of toluene. The toluene was dried, filtered and evaporated to a foam. The foam was dissolved in ethanol and converted to the HCl salt by the addition of concentrated HCl. The solvent was removed and the residue azeotroped first with ethanol then 1:1 ethanol-toluene followed by toluene. This residual material was crystallized from methanol-ethyl acetate to give 1.86 g (52%) of *33*, mp 165—177°. Recrystallization from the same solvent pair gave pure *33*, mp 174.5—177°.

| | | | | | | | | |
|---|---|---|---|---|---|---|---|---|
| *Anal.* Calcd. for $C_{22}H_{29}NO_2$·HCl | : | C, | 70.29; | H, | 8.04; | N, | 3.73. |
| Found | : | C, | 70.30; | H, | 8.07; | N, | 3.59. |

K. 17-Cyclopropylmethyl-7β,8α-dimethyl-3-methoxyisomorphinan-6-one Hydrochloride (34) (TR—5331).

A mixture of *31* (1.84 g, 6.1 mmol) in DMF (35 ml) containing sodium bicarbonate (0.78 g, 9.2 mmol) and cyclopropylmethyl bromide (1.00 g, 7.4 mmol) was heated at 100° under an argon atmosphere for 17 hours. Processing as above for *33* gave a syrup which was converted to the HCl salt and crystallized from methanol -ethyl acetate to give 1.68 g (71%) of pure *34*, mp >265°.

| | | | | | | | |
|---|---|---|---|---|---|---|---|
| *Anal.* Calcd. for $C_{23}H_{31}NO_2$·HCl | : | C, | 70.84; | H, | 8.27; | N, | 3.59. |
| Found | : | C, | 71.07; | H, | 8.34; | N, | 3.81. |

L. 17-Cyclobutylmethyl-7β,8α-dimethyl-3-methoxyisomorphinan-6-one Hydrochloride (35) (TR—5332).

Prepared from compound *31* (1.84 g, 6.1 mmol) and cyclobutylmethyl bromide (1.10 g, 7.4 mmol) in DMF (35 ml) containing sodium bicarbonate (0.78 g, 9.2 mmol) at 100° overnight. Workup in the usual fashion gave 1.96 g (86%) of the free base of *38* as a glass which was converted to the HCl salt and crystallized from methanol -ethyl acetate to give pure *35*, mp >265°.

*Anal.* Calcd. for $C_{24}H_{33}NO_2$·HCl : C, 71.35; H, 8.48; N, 3.47.
Found : C, 71.13; H, 8.48; N, 3.36.

M. 17-Cyclobutylmethyl-8α-ethyl-3-methoxy-7β-methylisomorphinan-6-one Hydrochloride (36).

Compound *35* (3.70 g, 11.8 mmol), sodium bicarbonate (2.48 g, 29.5 mmol) and cyclobutylmethyl bromide (2.11 g, 14.2 mmol) in DMF (65 ml) were reacted in the usual fashion for 6.5 hours. Workup as previously described gave 3.84 g (85%) of the free base of *36* as a glass. This was converted to the HCl salt which was twice recrystallized from methanol-ethyl acetate to give pure *36*, mp >265°.

*Anal.* Calcd. for $C_{25}H_{35}NO_2$·HCl : C, 71.83; H, 8.68; N, 3.35.
Found : C, 71.43; H, 8.89; N, 3.22.

N. 17-Cyclopropylmethyl-3-hydroxy-7β-methyl-isomorphinan-6-one Hydrobromide (37) (TR—5225).

A mixture of *33* (4.2 g) and 48% HBr (25 ml) was refluxed in an oil bath preheated to 140° for 20 minutes. The resulting suspension was cooled and filtered to give 3.31 g of white crystals. The crystals were converted to the free base and further purified by chromatography to give the free base of *37* as a foam. This foam was converted to the HBr salt in a manner analagous to that used for the HCl salts. Crystallization from aqueous ethanol gave pure *37*, mp >265°.

*Anal.* Calcd. for $C_{21}H_{27}NO_2$·HBr : C, 62.07; H, 6.95; N, 3.45.
Found : C, 62.15; H, 6.99; N, 3.36.

O. 17-Cyclopropylmethyl-7β,8α-dimethyl-3-hydroxy-isomorphinan-6-one Hydrobromide (38) (TR—5343).

A mixture of *34* (free base, 2.44 g) and 48% HBr (25 ml) was refluxed for 15 minutes. The solution was cooled and the crystalline *38* (2.21 g, 76%) collected. The crystals, mp >270°, were purified by boiling with aqueous methanol.

*Anal.* Calcd. for $C_{22}H_{29}NO_2$·HBr : C, 62.86; H, 7.19; N, 3.33.
Found : C, 62.53; H, 7.15; N, 3.06.

P. 17-Cyclobutylmethyl-8α-ethyl-3-hydroxy-7β-methyl-isomorphinan-6-one Hydrochloride (39) (TR—5370).

A mixture of *36* (2.1 g) and 48% HBr (20 ml) was refluxed for 20 minutes then processed as above and chromatographed to give 873 mg (43%) of the free base of *39*. This was converted to the HCl salt which crystallized from methanol-ethyl acetate to give pure *39*, mp >270°.

*Anal.* Calcd. for $C_{24}H_{33}NO_2$·HCl : C, 71.35; H, 8.48; N, 3.47.
Found : C, 71.05; H, 8.52; N, 3.39.

## EXAMPLE III
## PHARMACOLOGICAL EVALUATION

The compounds whose preparation is disclosed in the foregoing examples were screened to determine the following biological activities:

(A) Analgesic effects upon mice (acetic acid writhing test).

(B) Narcotic antagonist activity in rats (modified rat tail flick test).

### TEST A.
### ACETIC ACID MOUSE WRITHING TEST

The analgestic effects of test compounds were determined in mice by use of the acetic acid writhing test described by B.J.R. Whittle, Brit. J. Pharmacol., *22*:246 (1964). In this test at least three groups of five male CD—1 mice (18—22 g) each were given subcutaneous doses of the test drug dissolved in either distilled water or distilled water acidified with HCl depending on the solubility of the compound. In all cases, 0.4 milliliter of a 0.5% V/V acetic acid in distilled water solution was administered intraperitoneally 15 minutes post drug. The number of writhes in a 20 min. interval beginning 5 minutes after the acetic acid injection were determined and compared with the number of writhes in control groups which had received only acetic acid.

Percent inhibition of writhing was calculated as:

$$\%\ \text{inhibition} = \frac{\text{No. Control writhes} - \text{No. treated writhes}}{\text{No. control writhes} \times 100}$$

The $ED_{50}$ dose, i.e., the dose required to reduce the number of writhes by 50%, was determined graphically from a plot of % inhibition as a probit versus log dose. Confidence limits of 95% were calculated on the basis of those results falling in the range 16—84% inhibition. See Litchfield, J. T. and Wilcoxon, F., J. Pharmacol. Exp. Ther., *96,* 99—113, (1949).

TEST B.

EVALUATION OF NARCOTIC ANTAGONIST ACTIVITY

The narcotic antagonist effects of test compounds were determined by a modification of the rat tail flick procedure of Harris and Pierson (J. Pharmacol. Exp. Ther. 143:141 [1964]).

Male albino Wistar rats (100—120 g) were used for this study. A rat's tail is so placed so as to cover a photocell. Heat is applied by a lamp in a reflector with a timer being connected to the lamp and photocell so that the timer goes on when the light is turned on and is turned off when the photocell is uncovered. A rheostat, incorporated into a heating lamp is used to adjust the intensity of the light falling on the tail of the rat such that the rat's control reaction time is from two to four seconds. Animals with a control reaction time outside this range are rejected. The rheostat adjustment is made only if a significant proportion (more than 2 out of every 10 rats) of the reaction times are outside the range of two to four seconds. Groups of five rats were used each time, and two control times were determined at 60 and 30 minutes prior to subcutaneous injection of the drug. A ten second cutoff time is employed; if the rat does not flick its tail in 10 seconds it is removed from the heat source.

Thirty minutes after the last control run the test drug was given intraperitoneally. This was followed ten minutes later by an $ED_{80}$ dose of morphine subcutaneously. The animals were retested at 20 minutes after the morphine injection. Control animals were given vehicle and morphine only. The data were calculated as follows:

$$\% \text{ Effect (E)} = \frac{[\text{MRT* (Treated)} - \text{MRT (Control)}] \times 100}{10 - \text{MRT (Control)}}$$

$$\% \text{ Antagonism} = \frac{[\text{E(morphine controls)} - \text{E(drug treated)}] \times 100}{\text{E (morphine control)}}$$

*MRT is defined as mean reaction time.

The data were plotted on log-probit paper and $AD_{50}$ values, i.e., the dose required to inhibit the morphine effect by 50% within 95% confidence limits, were determined by the method of Litchfield and Wilcoxon.

The results of these experiments are set out in Table I where $R_1$, $R_2$, $R_3$ and $R_4$ refer to the preceding Formula I for the compounds of the present invention. In the column under $R_2$, CBM stands for cyclobutylmethyl, CPM for cyclopropylmethyl. For purposes of this table, IA is intended to mean "inactive" at the dose indicated.

TABLE I

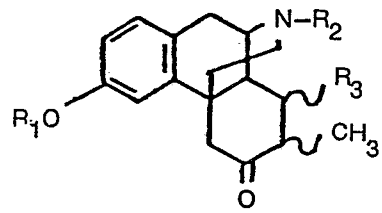

| Compound | Ex. | B/C | $R_1$ | $R_2$ | $R_3$ | 7—$CH_3$ | $ED_{50}$ | $AD_{50}$ |
|---|---|---|---|---|---|---|---|---|
| TR—5343 | II O | *trans* | H | CPM | α-$CH_3$ | β-$CH_3$ | 1.89 | 0.43 |
| TR—5225 | II N | *trans* | H | CPM | H | β-$CH_3$ | 0.72** | 0.48 |
| TR—5333 | I U | *Cis* | H | CBM | β-ethyl | α-$CH_3$ | 3.3 | 1.95 |
| TR—5195 *** | I T | *Cis* | H | CBM | β-$CH_3$ | α-$CH_3$ | 0.78 | 2.0 |
| TR—5177 | I R | *Cis* | H | CPM | H | α-$CH_3$ | 0.87 | 5.2 |
| TR—5188 | I O | *Cis* | $CH_3$ | CPM | β-$CH_3$ | α-$CH_3$ | 1.03 | 4.7 |
| TR—5205 | II J | *trans** | $CH_3$ | CPM | H | β-$CH_3$ | >15 | 1.2 |
| TR—5331 | II K | *trans** | $CH_3$ | CPM | α-$CH_3$ | β-$CH_3$ | >10 | >10 |
| TR—5332 | II L | *trans** | $CH_3$ | CBM | α-$CH_3$ | β-$CH_3$ | >10 | >10 |
| TR—5370 | II P | *trans** | H | CBM | α-ethyl | β-$CH_3$ | IA@10 | >10 |
| TR—5196 | I S | *Cis** | H | CPM | β-$CH_3$ | α-$CH_3$ | 7.8 | 0.12 |

* reference compounds
** short acting
*** novel action in charcoal meal test upon attempted naloxone reversal (TR—5177) which suggests that this compound would have longer duration of action than would be expected for a compound of this type.

## Claims

1. 7-methyl and 7-methyl-8-lower alkyl substituted B/C *cis* or *trans* morphinan-6-one compounds characterized by the structural formula:

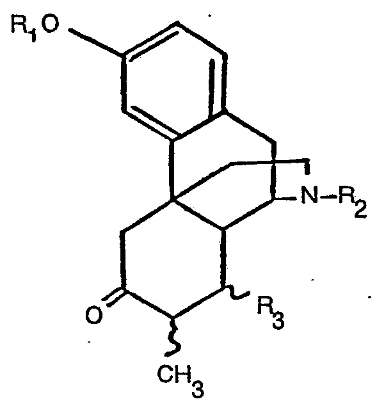

wherein

$R_1$ is H or methyl, $R_2$ is cyclopropylmethyl or cyclobutylmethyl and $R_3$ is H, methyl or ethyl provided that:

A. When the molecule is in the B/C *cis* configuration, the 7-methyl group is in the α-configuration; and

i. when $R_2$ is cyclobutylmethyl, $R_1$ is H and $R_3$ is β-ethyl or β-methyl, and
ii. when $R_2$ is cyclopropylmethyl and $R_1$ is H, $R_3$ is H, and
iii. when $R_2$ is cyclopropylmethyl and $R_1$ is methyl, $R_3$ is β-methyl;

B. When the molecule is in the B/C *trans* configuration, the 7-methyl group is in the $\beta$-configuration, $R_1$ is H, $R_2$ is cyclopropylmethyl and $R_3$ is H or $\alpha$-methyl.

2. The compounds of Claim 1 in the form of their organic or inorganic acid addition salts.

3. A compound as defined by Claim 1 wherein $R_1$ is H, $R_2$ is cyclopropylmethyl, $R_3$ is $\alpha$-methyl and which is in the B/C *trans* configuration.

4. A compound as defined by Claim 1 wherein $R_1$ is H, $R_2$ is cyclopropylmethyl, $R_3$ is H and which is in the B/C *trans* configuration.

5. A compound as defined by Claim 1 wherein $R_1$ is H, $R_2$ is cyclobutylmethyl, $R_3$ is $\beta$-ethyl and which is in the B/C *cis* configuration.

6. A compound as defined by Claim 1 wherein $R_1$ is H, $R_2$ is cyclobutylmethyl, $R_3$ is $\beta$-methyl and which is in the B/C *cis* configuration.

7. A compound as defined by Claim 1 wherein $R_1$ is H, $R_2$ is cyclopropylmethyl, $R_3$ is H and which is in the B/C *cis* configuration.

8. A compound as defined by Claim 1 wherein $R_1$ is methyl $R_2$ is cyclopropylmethyl $R_3$ is $\beta$-methyl and which is in the B/C *cis* configuration.

9. A therapeutic composition which comprises an effective amount of a compound as claimed in claim 1.

10. A process for the manufacture of 7-methyl and 7-methyl-8-lower alkyl substituted B/C cis or trans morphinan-6-one compounds characterized by the structural formula:

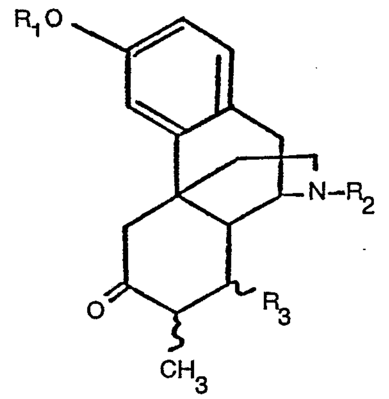

wherein

$R_1$ is H or methyl, $R_2$ is cyclopropylmethyl or cyclobutylmethyl and $R_3$ is H, methyl or ethyl provided that:

A. When the molecule is in the B/C *cis* configuration, the 7-methyl group is in the $\alpha$-configuration; and

i. when $R_2$ is cyclobutylmethyl, $R_1$ is H and $R_3$ is $\beta$-ethyl or $\beta$-methyl, and

ii. when $R_2$ is cyclopropylmethyl and $R_1$ is H, $R_3$ is H, and

iii. when $R_2$ is cyclopropylmethyl and $R_1$ is methyl, $R_3$ is $\beta$-methyl;

B. When the molecule is in the B/C *trans* configuration, the 7-methyl group is in the $\beta$-configuration, $R_1$ is H, $R_2$ is cyclopropylmethyl and $R_3$ is H or $\alpha$-methyl, characterized in

a) reacting thebaine with lithium dimethyl cuprate in a halogenated hydrocarbon or aromatic hydrocarbon solvent to convert it to 3,6-dimethoxy-7$\beta$,17-dimethyl-4-hydroxy-5,6,8,14-tetrahydro-morphinane;

b) converting the reaction product of a) to its 4-phenyl ether by reaction with bromobenzene in the presence of an acid acceptor and copper powder;

c) cleaving the 4-phenoxy group from the reaction product of step b) by reacting it with sodium in an appropriate solvent;

d) hydrolyzing the reaction product of step c) with an appropriate acid to convert it to an isomeric mixture of 7,8-didehydro-7,17-dimethyl-3-methoxymorphinan-6-one and 7,8-didehydro-7,17-dimethyl-3-methoxy-isomorphinan-6-one;

e) optionally separating the morphinan and isomorphinan isomers formed in step d) and carrying out the following reaction steps on the individual isomers or the isomeric mixture;

f) reducing the 7,8-double bond in the reaction product of step d) or e) by treatment with hydrogen over a palladium-charcoal catalyst or

g) alternatively to f) alkylating the products obtained in steps d) or e) with lithium dimethyl copper or lithium divinyl copper; and

h) removing the 17-methyl group and introducing a cycloalkylmethyl group at this position.

**Revendications**

1. B/C cis ou trans morphinanones-6-méthyl-7 et méthyl-7 alkyle inférieur-8-substituées, caractérisées par la formule développée:

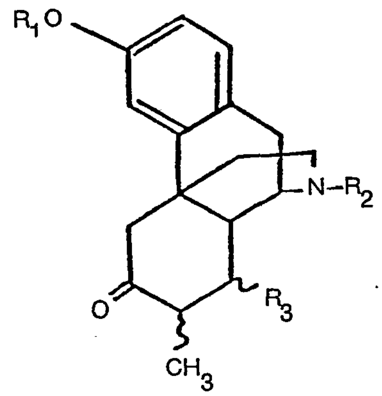

où $R_1$ représente H ou un radical méthyle, $R_2$ représente un radical cyclopropylméthyle ou cyclobutylméthyle et $R_3$ représente H ou un radical méthyle ou éthyle, sous réserve que:

A. lorsque la molécule a la configuration B/C cis, le radical méthyl-7 a la configuration $\alpha$; et

1. lorsque $R_2$ représente le radical cyclobutylméthyle, $R_1$ représente H et $R_3$ représente le radical $\beta$-éthyle ou $\beta$-méthyle, et

2. lorsque $R_2$ représente le radical cyclopropylméthyle et $R_1$ représente H, $R_3$ représente H, et

3. lorsque $R_2$ représente le radical cyclopropylméthyle et $R_1$ représente le radical méthyle, $R_3$ représente le radical $\beta$-méthyle;

B. lorsque la molécule a la configuration B/C trans, le radical méthyl-7 a la configuration $\beta$, $R_1$ représente H, $R_2$ représente un radical cyclopropylméthyle et $R_3$ représente H ou un radical $\alpha$-méthyle.

2. Composés selon la revendication 1, sous forme de leurs sels d'addition d'acides organiques ou minéraux.

3. Composé selon la revendication 1, où $R_1$ représente H, $R_2$ représente le radical cyclopropylméthyle, $R_3$ représente un radical $\alpha$-méthyle et qui a la configuration B/C trans.

4. Composé selon la revendication 1, où $R_1$ représente H, $R_2$ représente le radical cyclopropylméthyle, $R_3$ représente H et qui a la configuration B/C trans.

5. Composé selon la revendication 1 où $R_1$ représente H, $R_2$ représente le radical cyclobutylméthyle, $R_3$ représente le radical $\beta$-éthyle et qui a la configuration B/C cis.

6. Composé selon la revendication 1, où $R_1$ représente H, $R_2$ représente le radical cyclobutylméthyle, $R_3$ représente un radical $\beta$-méthyle et qui a la configuration B/C cis.

7. Composé selon la revendication 1, où $R_1$ représente H, $R_2$ représente le radical cyclopropylméthyle, $R_3$ représente H et qui a la configuration B/C cis.

8. Composé selon la revendication 1, où $R_1$ représente le radical méthyle, $R_2$ représente le radical cyclopropylméthyle, $R_3$ représente le radical $\beta$-méthyle, et qui a la configuration B/C cis.

9. Composition thérapeutique qui comprend une quantité efficace d'un composé selon la revendication 1.

10. Procédé pour la préparation de B/C cis ou trans morphinanones-6-méthyl-7 et méthyl-7 alkyle inférieur-8-substituées, caractérisées par la formule développée:

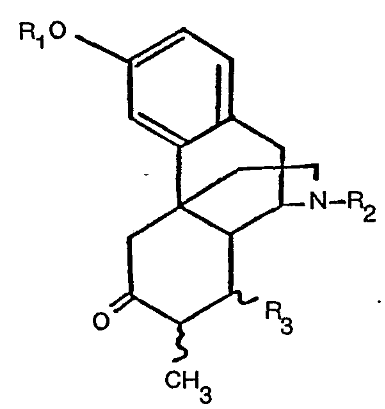

où $R_1$ représente H ou le radical méthyle, $R_2$ représente le radical cyclopropylméthyle ou cyclobutylméthyle et $R_3$ représente H ou un radical méthyle ou éthyle, sous réserve que:

A. lorsque la molécule a la configuration B/C cis, le radical méthyl-7 a la configuration $\alpha$; et

1. lorsque $R_2$ représente le radical cyclobutylméthyle, $R_1$ représente H et $R_3$ représente le radical $\beta$-éthyle ou $\beta$-méthyle, et

2. lorsque $R_2$ représente le radical cyclopropylméthyle et $R_1$ réprésente H, $R_3$ représente H, et

3. lorsque $R_2$ représente le radical cyclopropylméthyle et $R_1$ représente le radical méthyle, $R_3$ représente le radical $\beta$-méthyle;

B. lorsque la molécule a la configuration B/C trans, le radical méthyl-7 a la configuration $\beta$, $R_1$ représente H, $R_2$ représente le radical cyclopropylméthyle et $R_3$ représente H ou le radical $\alpha$-méthyle, caractérisé en ce qu'il consiste à:

a) faire réagir la thébaïne avec le cuprate de lithium-diméthyle dans un solvant constitué d'un hydrocarbure halogéné ou d'un hydrocarbure aromatique pour la transformer en diméthoxy-3,6-diméthyl-7$\beta$,17 hydroxy-4 tétrahydro-5,6,8,14- morphinane;

b) transformer le produit réactionnel de a) en le dérivé phénoxy-4 par réaction avec le bromobenzène en présence d'un accepteur d'acide et de poudre de cuivre;

c) cliver le radical phénoxy-4 du produit réactionnel du stade b) en le faisant réagir avec du sodium dans un solvant approprié;

d) hydrolyser le produit réactionnel du stade c) avec un acide approprié pour le transformer en un mélange isomère de didéhydro-7,8 diméthyl-7,17 méthoxy-3 morphinanone-6 et de didéhydro-7,8 diméthyl-7,17 méthoxy-3 isomorphinanone-6;

e) éventuellement séparer les isomères de type morphinane et isomorphinane formés dans le stade d) et effectuer les stades réactionnels suivants sur les isomères individuels ou le mélange des isomères;

f) réduire la double liaison 7,8 du produit réactionnel du stade d) ou e) par traitement avec de l'hydrogène sur un catalyseur constitué de carbon palladié ou

g) au lieu de f), alkyler les produits obtenus dans les stades d) ou e) avec du lithium-diméthyl-cuivre ou du lithium-divinyl-cuivre; et

h) éliminer le radical méthyl-17 et introduire un radical cycloalkylméthyle en cette position.

**Patentansprüche**

1. 7-Methyl- und 7-Methyl-8-niedrigalkyl-substituierte B/C cis- oder trans-morphinan-6-on-Verbindungen, gekennzeichnet durch die Strukturformel

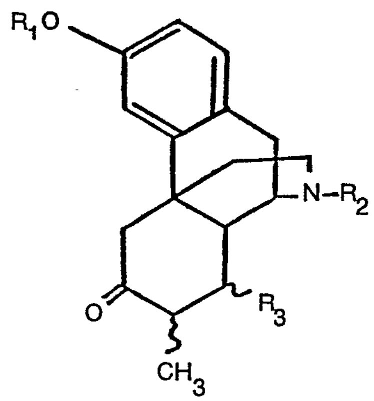

worin $R_1$ H oder Methyl, $R_2$ Cyclopropylmethyl oder Cyclobutylmethyl und $R_3$ H, Methyl oder Ethyl bedeuten, mit dem Proviso, dass

(A) wenn das Molekül in der B/C cis-Konfiguration vorliegt, ist die 7-Methylgruppe in der $\alpha$-Konfiguration und

(i) wenn $R_2$ Cyclobutylmethyl bedeutet, ist $R_1$ H und $R_3$ $\beta$-Ethyl oder $\beta$-Methyl, und

(ii) wenn $R_2$ Cyclopropylmethyl bedeutet, ist $R_1$ H und $R_3$ H, und

(iii) wenn $R_2$ Cyclopropylmethyl und $R_1$ Methyl bedeuten, ist $R_3$ $\beta$-Methyl;

(B) wenn das Molekül in der B/C trans-Konfiguration vorliegt, so liegt die 7$\beta$-Methylgruppe in der $\beta$-Konfiguration vor und $R_1$ ist H, $R_2$ ist Cyclopropylmethyl und $R_3$ ist H oder $\alpha$-Methyl.

2. Verbindungen gemäss Anspruch 1, in Form ihrer organischen oder anorganischen Säureadditionssalze.

3. Eine Verbindung gemäss Anspruch 1, worin $R_1$ H, $R_2$ Cyclopropylmethyl, $R_3$ $\alpha$-Methyl bedeuten und in welcher die B/C trans-Konfiguration vorliegt.

4. Eine Verbindung gemäss Anspruch 1, worin $R_1$ H, $R_2$ Cyclopropylmethyl, $R_3$ H bedeuten und welche in der B/C trans-Konfiguration vorliegt.

5. Eine Verbindung gemäss Anspruch 1, worin $R_1$ H, $R_2$ Cyclobutylmethyl, $R_3$ $\beta$-Ethyl bedeuten und welche in der B/C cis-Konfiguration vorliegt.

6. Eine Verbindung gemäss Anspruch 1, worin $R_1$ H, $R_2$ Cyclobutylmethyl, $R_3$ $\beta$-Methyl bedeuten und welche in der B/C cis-Konfiguration vorliegt.

7. Eine Verbindung gemäss Anspruch 1, worin $R_1$ H, $R_2$ Cyclopropylmethyl, $R_3$ H bedeuten und welche in der B/C cis-Konfiguration vorliegt.

8. Eine Verbindung gemäss Anspruch 1, worin $R_1$ Methyl, $R_2$ Cyclopropylmethyl, $R_3$ $\beta$-Methyl bedeuten und welche in der B/C cis-Konfiguration vorliegt.

9. Eine therapeutische Zusammensetzung, enthaltend eine wirksame Menge einer Verbindung gemäss Anspruch 1.

10. Verfahren zur Herstellung von 7-Methyl- und 7-Methyl-8-niedrigalkyl-substituierten B/C cis- oder transmorphinan-6-on-Verbindungen, gekennzeichnet durch die Strukturformel

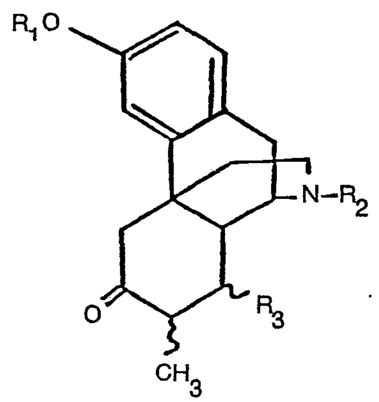

worin $R_1$ H oder Methyl, $R_2$ Cyclopropylmethyl oder Cyclobutylmethyl und $R_3$ H, Methyl oder Ethyl bedeuten, mit dem Proviso, dass

(A) wenn das Molekül in der B/C cis-Konfiguration vorliegt, die 7-Methylgruppe in der $\alpha$-Konfiguration vorliegt, und

(i) wenn $R_2$ Cyclobutylmethyl bedeutet, $R_1$ H und $R_3$ $\beta$-Ethyl oder $\beta$-Methyl bedeutet, und

(ii) wenn $R_2$ Cyclopropylmethyl bedeutet, $R_1$ H und $R_3$ H bedeuten, und

(iii) wenn $R_2$ Cyclopropylmethyl und $R_1$ Methyl bedeuten, $R_3$ $\beta$-Methyl ist;

(B) wenn das Molekül in der B/C trans-Konfiguration vorliegt, die 7-Methylgruppe in der $\beta$-Konfiguration vorliegt, $R_1$ H, $R_2$ Cyclopropylmethyl und $R_3$ H oder $\alpha$-Methyl bedeutet, dadurch gekennzeichnet, dass man

(a) Thebain mit Lithiumdimethylcuprat in einem halogenierten Kohlenwasserstoff- oder aromatischen Kohlenwasserstoff-Lösungsmittel umsetzt, unter Bildung von 3,6-Dimethoxy-7$\beta$,17-dimethyl-4-hydroxy-5,6,8,14-tetrahydromorphinan;

(b) das Reaktionsprodukt von (a) durch Umsetzen mit Brombenzol in Gegenwart eines Säureakzeptors und von Kupferpulver in den 4-Phenylether überführt,

(c) die 4-Phenoxygruppe aus dem Reaktionsprodukt der Stufe (b) durch Umsetzen mit Natrium in einem geeigneten Lösungsmittel abspaltet,

(d) das Reaktionsprodukt aus Stufe (c) mit einer geeigneten Säure hydrolysiert und dadurch in ein Isomerengemisch von 7,8-Didehydro-7,17-dimethyl-3-methoxymorphinan-6-on und 7,8-Didehydro-7,17-dimethyl-3-methoxy-isomorphinan-6-on umwandelt,

(e) gewünschtenfalls die in Stufe (d) gebildeten Morphinan- und Isomorphinanisomere trennt und die nachfolgenden Reaktionsstufen mit den einzelnen Isomeren oder mit dem Isomerengemisch vornimmt,

(f) die 7,8-Doppelbindung in dem Reaktionsprodukt der Stufe (d) oder (e) durch Behandeln mit Wasserstoff über Palladium-auf-Holzkohle-Katalysator reduziert, oder

(g) alternativ zu (f) die in den Stufen (d) oder (e) erhaltenen Produkte mit Lithiumdimethylkupfer oder Lithiumdivinylkupfer alkyliert und

(h) die 17-Methylgruppe entfernt und in deren Stellung eine Cycloalkylmethylgruppe einführt.